# EUROPEAN PATENT SPECIFICATION

(11) **EP 0 541 617 B1**
(45) Date of publication and mention of the grant of the patent: **04.06.1997**
(21) Application number: 91913695.2
(22) Date of filing: 15.07.1991
(51) Int. Cl.: C07D 295/08, C07D 261/06, C07D 263/08, C07D 271/06, C07D 333/06

(54) **1,4-DISUBSTITUTED PIPERAZINES**
1,4-DISUBSTITUIERTE PIPERAZINE
PIPERAZINES 1,4-BISUBSTITUEES

(30) Priority: 26.07.1990 DK 1785/90
(43) Date of publication of application: 19.05.1993
(73) Proprietor: NOVO NORDISK A/S, 2880 Bagsvaerd (DK)
(72) Inventor: HOHLWEG, Rolf, DK-3490 Kvistgaard (DK); GUDDAL, Erling, DK-2605 Brondby (DK); NIELSEN, Erik, Bardrum, DK-3500 Vaerlose (DK)
(86) International application number: DK9100205
(87) International publication number: WO9201679

(56) References cited:
- EP-A- 0 243 903
- EP-A- 0 254 627
- CH-A- 634 313
- DE-A- 2 621 082
- DE-A- 3 726 068
- FR-A- 2 276 824
- US-A- 2 988 551
- US-A- 3 558 631
- US-A- 4 973 591

## Description

This invention concerns novel diarylalkoxyalkylpiperazines useful for their inhibitory activity at dopamin uptake sites, a method of preparing the same, pharmaceutical compositions containing them and their use for treating mental disorders as e.g. depression and other CNS-related diseases as Parkinson's disease.

Benzhydrylpiperazines, where the benzhydryl group is connected to the nitrogen of a piperazine over a -O-(CH₂)n- moiety, are known from the German patent publication No. DOS 2719246, where spasmolytic and antiemetic activity is claimed while EP patent appl. Nos. 157420 and 285219 claim lipoxygenase inhibiting and sleep improving properties respectively. Furthermore, in US-patent No. 4766215 and European patent application No. 0254627 similar benzhydrylethers are described as antihistaminic compounds; German patent publication No. DE 3726068 claims calcium antagonistic activity for compounds with the aforementioned general structure. Other compounds with the mentioned general structure are described in US 3558631 as having mild tranquilizing and antidepressant activity and are also described in European patent application Nos. 0243903, 0243904, 0243905, in CH-A-634313, in GB Patent No. 1545094 and Dutch patent appl. No. NL 8202636 and claimed to be useful in case of degeneration or hypofunction of the dopaminergic system.

In US-patent No. US 4,096,259, similar compounds bearing polar carboxyl- or alkoxycarbonyl groups are described as central stimulants, however, no dopaminergic activity is claimed or evident from the patent description.

Generally the compounds mentioned in the different patent specifications vary greatly with reference to the substitution of the second piperazine nitrogen and thus may give rise to the great variety in pharmacological activity.

It has now unexpectedly been found that by introducing a substituent containing certain polar groups in the 4-position of the aforementioned piperazine compounds, new and novel compounds with greatly improved in vivo activity can be obtained. It is expected that such substances possess antidepressant, antiparkinson; antipsychotic, antispastic, memory enhancing, as well as other similarly useful therapeutic effects in diseases in which a dopaminergic deficit is implicated. The substances may also have beneficial effects against drug craving or drug abuse.

The compounds of the invention have the general formula I: 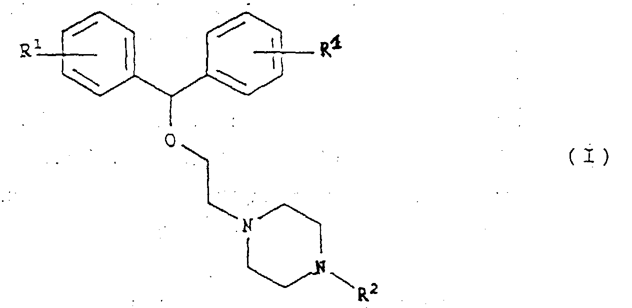 wherein R¹ is halogen, methoxy, C₁₋₆-alkyl or trifluoromethyl, and R² is a straight or branched C₁₋₈-alkyl or C₃₋₈-alkenyl, which in any position may be substituted with hydroxy-, keto- hydroxy imino-groups leading to a stable tertiary amine, but is terminally substituted with cyano, optionally substituted thienyl, furyl, where the optional substitution is represented by C₁₋₆-alkyl or phenyl, provided that when cyano is the only substitutent in R², R² must contain at least five carbon atoms besides CN, and pharmaceutically acceptable acid addition salts thereof.

The compounds of formula I may exist as geometric and optical isomers and all isomers and mixtures thereof are included herein. Isomers may be separated by means of standard methods such as chromatographic techniques or fractional crystallization of suitable salts.

Pharmaceutically acceptable acid addition salts of compounds of formula I include those derived from inorganic or organic acids such as hydrochloric, hydrobromic, sulfuric, phosphoric, acetic, lactic, maleic, phthalic and fumaric acid.

The invention also relates to a method of preparing the above mentioned compounds. This method comprises:
a) reacting a compound of formula II 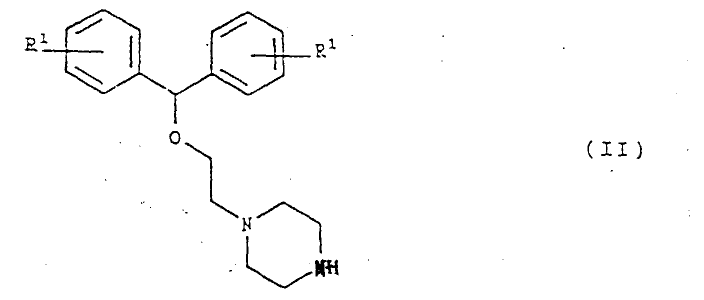 where R¹ has the meaning defined above with a reactive reagent containing the group R² as defined above, or groups that may be converted to group R² by generally known methods.
   Generally known methods for alkylating secondary amines are for example:
   1) reaction of the monosubstituted piperazine of formula II with an alkyl halide or tosylate.
   2) addition of the monosubstituted piperazine compound of formula II to a reactive double bond or epoxide.
   3) acylation of the monosubstituted piperazine compound of formula II by known methods followed by reduction to yield the desired tertiary amine of formula I.
b) reacting a compound of formula III 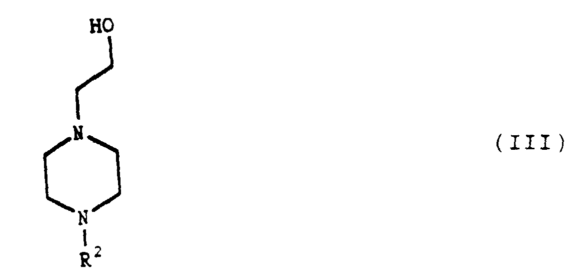 prepared by alkylation of 1-(2-hydroxyethyl)-piperazine according to one of the aforementioned methods, and thereafter combining this disubstituted piperazine with a compound of formula V 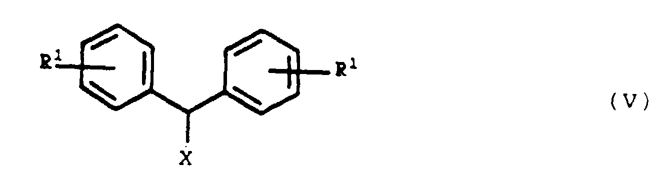 where R¹ has the meaning mentioned above, and X means OH or halogen, applying standard methods of ether synthesis, consisting in a condensation reaction with the removal of water or hydrogen halide.
c) reacting a compound of the formula IV 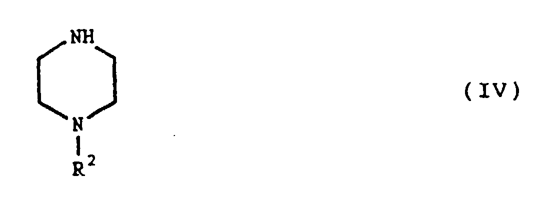 prepared by alkylating piperazine according to generally well known methods such as described in section a) and b) above for introducing the group R², with an optionally substituted diarylmethoxyethyl derivative of formula VI 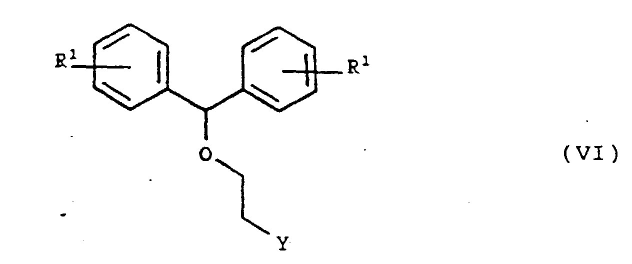 where R¹ has the meaning as defined above and Y means halogen to obtain the desired compound of formula I.

The biological properties of the compounds according to the invention can be illustrated in the following way:

### Biochemistry

### Test 1

The compounds were tested for their ability to inhibit the binding of 3H-GBR 12935 (the des(bis(4-fluoro) analog of GBR 12909)to the DA-uptake complex in a striatal membrane preparation following the method described by Andersen (Eur.J.Pharmacol. 166: 493-504, 1989).

### Pharmacology

The compounds were also tested for their ability to increase the motility of mice following intravenous administration using the method outlined below.

### Test 2

### Subjects

Male NMRI mice are used (20 ± 2 g). The animals are housed 20-30 per cage under constant temperature (20 ± 1°C) and relative humidity (40-60%). The animals are brought into the experimental room in the afternoon, the day before they undergo testing.

### Methods

The experimentally-naive, uninjected mice are acclimatized by being placed in a plexiglass box (WLH: 20 x 20 x 38 cm), four per box, for a 120 min period, and then injected with the test compound whereafter they are replaced in the plexiglass box.

The plexiglass box is equipped with a frame of photocells (spaced equidistantly) which are situated so as to detect locomotor behaviour of the animals (1 cm above the floor). The photocell chamber is housed in an sound-insulated, dimly-lit, ventilated chest. As a measure of exploratory behaviour, the number of photocell crossings in a 360 min period is detected using a minicomputer. Testing is made between 7:00 and 17.00 h.

### Drug Testing

Drugs are injected i.v. simultaneously with start of testing; N = 4/dose; 4-5 doses of test drug is given in a volume of 10 ml/kg.

### Results

computer programmed log-probit methods are used to generate an ED₅₀ in mg/kg using as minimum the control result, and as maximum values are used 7700 (this latter value has been experimentally found as the maximum motility counts after d-amphetamine in a 20 min period).

The test result is the lowest ED₅₀ value determined at a 20 min interval during the 360 min test period.

The present invention relates to the increase in the ratio between in vitro inhibition of 3H-GBR 12935 binding and the in.vivo activity in the increasing motility. Thus, a reference compound such as GBR 12909 (1-(2-(bis(4-fluorophenyl)-methoxy)-ethyl)-4-(3-phenyl-propyl) piperazine disclosed in CH-A-634313 and in Eur. J. Pharmacol. 166:493-504, 1989) had a ratio of only 3.2, whereas for the compound described in example 4A the ratio was increased to 7.5. Thus, this compound had higher in vivo activity than can be expected when comparing with GBR 12909.

Test results are shown in the following table I:

**TABLE 1**

| Compound | Test 1(nm) | Test 2(mg/kg) | Ratio |
|---|---|---|---|
| Example 4A | 18 | 2.4 | 7.5 |
| GBR 12909 | 7.5 | 2.3 | 3.2 |

The pharmaceutical preparations or compositions comprising the compounds of the invention may be administered to humans or animals by oral or parenteral route.

An effective amount of the active compound or a pharmaceutically-acceptable salt thereof may be determined in accordance with the usual factors, such as the nature and severity of the condition and the weight of the mammal requiring treatment.

Conventional excipients are such pharmaceutically-acceptable organic or inorganic carrier substances suitable for parenteral or enteral application which do not deleteriously react with the active compounds.

Examples of such carriers are water, salt solutions, alcohols, polyethylene glycols, polyhydroxyethoxylated castor oil, gelatine, lactose, amylose, magnesium stearate, talc, silicic acid, fatty acid monoglycerides and diglycerides, pentaerythritol fatty acid esters, hydroxymethylcellulose, and polyvinylpyrrolidone.

The pharmaceutical preparations can be sterilized and mixed, if desired, with auxiliary agents, such as lubricants, preservatives, stabilizers, wetting agents, emulsifiers, salt for influencing osmotic pressure, buffers and/or coloring substances and the like, which do not deleteriously react with the active compounds.

Injectable solutions or suspensions, preferably aqueous solutions with the active compound dissolved in polyhydroxylated castor oil, are particularly suitable for parenteral administration.

Ampoules are convenient unit dosage forms.

Tablets, dragees, or capsules containing talc and/or a carrier or binder or the like are particularly suitable for oral administration. The carrier preferably is lactose and/or corn starch and/or potato starch.

A syrup, elixir, or the like can be used in the cases where a sweetened vehicle can be employed or is desired.

Generally, the compounds of this invention are dispensed in unit dosage form comprising 50-200 mg of active ingredient in or together with a pharmaceutically-acceptable carrier per unit dosage.

The dosage of the compounds according to this invention is 1-500 mg/day, e.g., about 100mg per dose, when administered to patients, e.g., humans, as a drug.

A typical tablet which may be prepared by conventional tabletting techniques contains:

| Core: | |
|---|---|
| Active compound (as free compound or salt thereof) | 100 mg |
| Colloidal silicon dioxide (Aerosil®) | 1.5 mg |
| Cellulose, microcryst. (Avicel®) | 70 mg |
| Modified cellulose gum (Ac-Di-Sol®) | 7.5 mg |
| Magnesium stearate | 1 mg |

| Coating: | | |
|---|---|---|
| HPMC | approx. | 9 mg |
| *Mywacett® 9-40 T | approx. | 0.9 mg |

| | | |
|---|---|---|
| * Acylated monoglyceride used as plasticizer for film-coating | | |

The compounds of the invention, together with a conventional adjuvant, carrier, or diluent, may be placed into the form of pharmaceutical compositions and unit dosages thereof, and in such form may be employed as solids, such as tablets or filled capsules, or liquids, such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical composition and unit dosage forms thereof may comprise conventional ingredients in conventional proportions, with or without additional active compounds or principles, and such unit dosage forms may contain any suitable effective DA-uptake inhibitory amount of the active ingredient commensurate with the intended daily dosage range to be employed. Tablets containing fifty (50) milligrams of active ingredient or, more broadly, ten (10) to two hundred (200) milligrams, per tablet, are accordingly suitable representative unit dosage forms.

Due to their high degree of DA-uptake inhibitory activity and their low toxicity, together presenting a most favorable therapeutic index, the compounds of the invention may be administered to a subject, e.g., a living animal body, in need of such treatment, elimination, alleviation, or amelioration of an indication which is sensitive to a change in the neuronal reuptake of dopamine, often preferably in the form of a non-toxic acid addition salt thereof, e.g. a hydrohalide, especially hydrochloride and hydrobromide, or a sulfate, nitrate, phosphate and the like, or an organic salt as acetate, propionate, lactate, malonate, succinate, maleate, fumarate, citrate and the like, concurrently, simultaneously, or together with a pharmaceutically- acceptable carrier or diluent, especially and preferably in the form of a pharmaceutical composition thereof, whether by oral, rectal, or parenteral (including subcutaneous) route, in an effective amount. Suitable dosage ranges are 50-200 milligrams daily, preferably 50-100 milligrams daily, and especially 70-100 milligrams daily, depending as usual upon the exact mode of administration, form in which administered, the indication toward which the administration is directed, the subject involved and the body weight of the subject involved, and the preference and experience of the physician or veterinarian in charge. Such method of treating may be described as the treatment of an indication caused by or related to the dopamine-system in a subject in need thereof, which comprises the step of administering to the said subject a neurologically- or neurolepticallyeffective amount of a DA-uptake inhibitory compound of the invention.

The invention will now be described in further detail with reference to the following examples.

### EXAMPLE 1

### 1-(2-[bis-(4-fluorophenyl)methoxy]ethyl)-4-[3-(3-thienyl)prop-2-enyl]piperazine

To a solution of 1.7 g (0.0051 mol) 1-(2-[bis(4-fluorophenyl)methoxy]ethyl)piperazine, US pat. 1545094, in a mixture of 10 ml toluene and 10 ml pyridine, 0.86 g (0.0050 mol) 3-(3-thienyl)propenoyl chloride, dissolved in 5 ml toluene, were added dropwise at room temperature.

The mixture was refluxed for 0.5 h, cooled, diluted with 100 ml toluene and successively washed with a 1N sodium hydroxide solution, 2 portions of water and brine. Evaporation of the toluene phase in vacuo gave 2.46 g of the intermediate 1-(2-[bis(4-fluorophenyl)methoxy]ethyl)-4-[3-(3-thienyl)-1-oxo-2-propenyl]piperazine as a dark oil.

This was dissolved in 20 ml of dry diethyl ether and added dropwise to a suspension of 0.48 g (0.0127 mol) lithium aluminium hydride in 30 ml of dry diethyl ether. The mixture was then heated to reflux for 1 h, cooled and hydrolyzed with a mixture of water and tetrahydrofuran. The solids were filtered off and to the filtrate was added an excess of a solution of maleic acid in diethyl ether. The precipitate was collected by filtration and recrystallized from methanol/isopropanol. Yield 0.80 g (23%) of the title compound as the dimaleate salt. M.p. 182-85°C.

NMR (400 MHz) in DMSO-d₆ [ppm]: 2.6-3.5 (m) 10H; 3.56 (m) 4H; 5.55 (s) 1H; 6.15 (m) 5H; 6.75 (d) 1H; 7.20 (t) 4H; 7.40 (m) 5H; 7.57 (m) 2H.

| | | | |
|---|---|---|---|
| Calculated for C₃₄H₃₆F₂N₂O₉S: | C 59.47%, | H 5.28%, | N 4.08% |
| Found : | C 59.69%, | H 5.27%, | N 3,97% |

### EXAMPLE 2

### 1-(2-[bis(4-fluorophenyl)methoxy]ethyl)-4-[4-oxo-4-(2-thienyl)butyl]piperazine

A mixture of 1.67 g (0.005 mol) 1-(2-[bis(4-fluorophenyl)-methoxy]ethyl)piperazine, (US Pat. 1545094), 1.89 g (0.010 mol) 3-chloropropyl 2-thienyl ketone, 1.52 g (0.011 mol) potassium carbonate and 30 ml methylisobutyl ketone was stirred and heated to reflux for 45 h. The reaction mixture was then evaporated in vacuo. The residue was redissolved in dichloromethane (100 ml) and washed twice with water (100 ml). The organic layer was then separated and dried with sodium sulfate. Evaporation in vacuo left a brown sirup. This was redissolved in dry ether and precipitated as the dihydrochloride with gaseous hydrogen chloride. The precipitate was collected by filtration and recrystallized from isopropanol. White crystals, m.p. 185-88°C (0.98 g = 35%).

NMR (400 MHz) in DMSO-d₆ [ppm]: 2.03 (m) 2H; 3.0-4.2 (m) 16H; 5.60 (s) 1H; 7.18 (t) 4H; 7.27 (t) 1H; 7.50 (dd) 4H; 8.00 (d) 1H; 8.04 (d) 1H.

| | | | |
|---|---|---|---|
| Calculated for C₂₇H₃₂Cl₂F₂N₂O₂S: | C 58.17, | H 5.79, | N 5.02% |
| Found : | C 58.47, | H 5.91, | N 4.94% |

### EXAMPLE 3

### 1-(2-[bis(4-fluorophenyl)methoxy]ethyl)-4-[4-hydroxy-4-(2-thienyl)butyl]piperazine

100 mg (0.00264 mol) sodium borohydride was added to a stirred solution of 0.72 g (0.00149 mol) 1-(2-[bis(4-fluorophenyl)methoxy]ethyl)-4-[4-oxo-4-(2-thienyl)butyl]piperazine (as described in example 4) in 8 ml ethanol. The reaction mixture was refluxed for 1 h, and then cooled and evaporated in vacuo. The residue was redissolved in diethyl ether, washed twice with water and once with brine. The ether solution was dried with sodium sulfate and the filtered solution treated with gaseous hydrogen chloride in excess. The precipitate was recrystallized from methanol, yielding the title compound as the dihydrochloride, m.p. 192-93°C.

NMR (400 MHz) in DMSO-d₆ [ppm]: 1.7 (m) 4H; 3.0-4.2 (m) 15H; 4.80 (t) 1H; 5.60 (s) 1H; 7.0 (m) 2H; 7.2 (t) 4H; 7.4 (t) 1H; 7.5 (dd) 4H.

| | | | |
|---|---|---|---|
| Calculated for C₂₇H₃₄Cl₂F₂N₂O₂S: | C 57.96, | H 6.12, | N 5.01% |
| Found : | C 58.41, | H 6.30, | N 5.00% |

### EXAMPLE 4

### 4A

### 1-[2-(bis-(4-fluorophenyl)-methoxy)-ethyl]-4-(4-cyanobutyl)-piperazine (not forming part of the invention)

1.0 g (3 mM) of 1-[2-bis(4-fluorophenyl)-methoxy)-ethyl]-piperazine was dissolved in 10 ml of dry dimethylformamide, 731 mg (4.5 mM) of 5-bromovaleronitrile and 830 mg of potassium carbonate were added, and the stirred mixture heated to 100°C. After 45 min. at 100° the reaction was completed as monitored by TLC (SiO₂/MeOH). To the cooled mixture 50 ml of water and 25 ml of toluene were added, the aqueous phase was extracted three times with toluene, the combined organic phases were washed with water and evaporated to yield 1.3 g of yellow oil. This crude product was dissolved in ether, and 1.3 ml of a 4.7 N solution of HCI in ether was added. The crystalline precipitate was redissolved in water, washed twice with methylene chloride, pH adjusted to 12 with NaOH and the product was extracted with methylene chloride.

Reprecipitation from ether with HCl yielded 510 mg of white crystals, melting at 187-9°C, and showing H-NMR in accordance with the dihydrochloride of the title compound.

NMR-data (DMSO) ppm: 1.6 (m) 2H; 1.8 (m) 2H; 2.6 (t) 2H; 3.1-3.8 (m) 14H (+H₂O); 5.6 (s) 1H; 7.2 (t) 4H; 7.5 (dd) 4H.

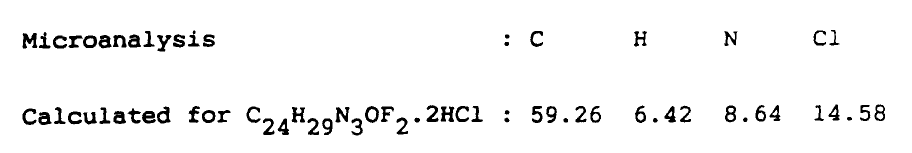

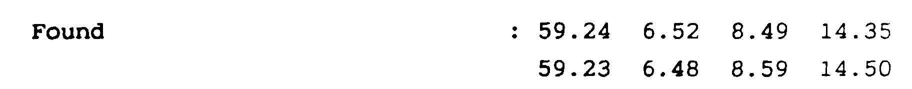

### 4B

### 1-[2-(bis(4-fluorophenyl)-methoxy)ethyl]4-(3-cyanopropyl)-piperazine (not forming part of the invention)

This compound was prepared by the general procedure as described in example no. 4A, starting from 4-bromobutyronitrile, and isolated as the dihydrochloride crystallized from ethanol as white crystals melting at 137-139°C, showing H-NMR spectrum in accordance with the title compound.

NMR-data (CDCl₃) ppm: 1.8 (m) 2H; 2.4-2.6 (m) 12H; 2.7 (t) 2H; 2.2 (m) 1H; 5.34 (s) 1H; 7.0 (t) 4H; 7.3 (dd) 4H; 3.55 (m) 3H.

| Microanalysis : | C | H | N | Cl |
|---|---|---|---|---|
| | | | | |
| Calculated for C₂₃H₂₇N₃OF₂.2HCl : | 63.36 | 6.47 | 9.64 | 8.13 |
| | | | | |
| Found : | 63.36 | 6.58 | 10.01 | 8.38 |
| | 63.08 | 6.50 | 9.95 | 8.46 |

In the same manner was prepared:

### 4C

### 1-(2-[Bis-(4-fluorophenyl)methoxy]ethyl)-4-(5-cyanopentyl)piperazine, dimaleate

White crystalline powder, m.p. 175-76°C.

NMR (400 MHz) in DMSO-d₆ (ppm): 1.37 (m) 2H; 1.60 (m) 4H; 2.6-3.4 (m) 14H; 3.53 (m) 2H; 5.53 (s) 1H; 6.18 (s) 4H; 7.18 (t) 4H; 7.38 (dd) 4H.

| | | | |
|---|---|---|---|
| Calculated for C₃₃H₃₈N₃O₉F₂: | C 60.08, | H 5.96, | N 6.37% |
| Found : | C 60.04, | H 6.00, | N 6.11% |

### 4D

### 1-(2-[Bis-(4-fluorophenyl)methoxy]ethyl)-4-(7-cyanoheptyl)piperazine, dimaleate

White crystalline powder, m.p. 164-65°C.

NMR (400 MHz) in DMSO-d₆ (ppm): 1.2-1.4 (m) 6H; 1.55 (m) 4H; 2.6-3.6 (m) 16H; 5.50 (s) 1H; 6.15 (s) 4H; 7.18 (t) 4H; 7.38 (dd) 4H.

| | | | |
|---|---|---|---|
| Calculated for C₃₅H₄₃N₃O₉F₂: | C 61.13, | H 6.30, | N 6.11% |
| Found : | C 60.85, | H 6.30, | N 5.94% |

## Claims (Claims for the following Contracting State(s): AT, BE, CH, LI, DE, DK, FR GB, IT, LU, NL, SE)

1. A disubstituted piperazine compound having the formula I: 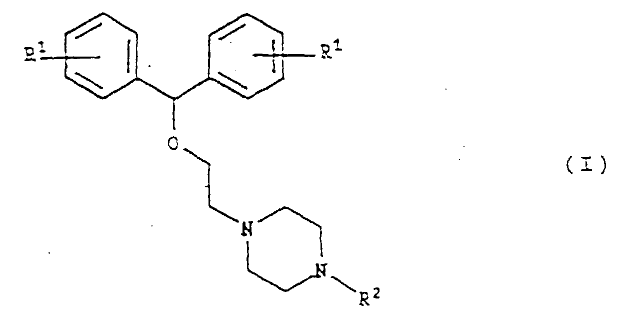 wherein R¹ is halogen, methoxy, C₁₋₆-alkyl or trifluoromethyl, and R² is a straight or branched C₁₋₈-alkyl or C₃₋₈-alkenyl, which in any position may be substituted with hydroxy-, keto-, or hydroxy imino groups leading to a stable tertiary amine, but is terminally substituted with cyano, optionally substituted thienyl, furyl, where the optional substitution is represented by C₁₋₆-alkyl or phenyl, provided that when cyano is the only substituent in R², R² must contain at least five carbon atoms besides CN, and pharmaceutically acceptable acid addition salts thereof.

2. A compound according to claim 1 selected from the group of
1-[2-(bis(-(4-fluorophenyl)-methoxy)-ethyl]-4-(5-cyanopentyl)-piperazine or
1-[2-(bis(-(4-fluorophenyl)-methoxy)-ethyl]-4-(7-cyanoheptyl)-piperazine.

3. A method of preparing a compound according to claim 1, CHARACTERIZED in
a) reacting a compound of formula II 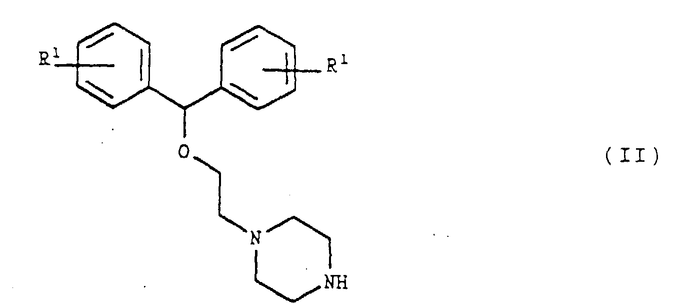 where R¹ has the meaning defined above with a reactive reagent containing the group R² as defined above, or groups that may be converted to group R²;
b) reacting a compound of formula III 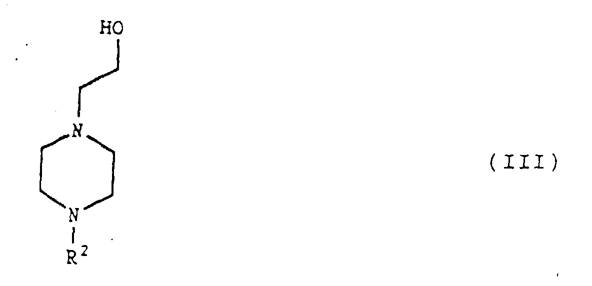 prepared by alkylation of 1-(2-hydroxyethyl)-piperazine and thereafter combining this disubstituted piperazine with a compound of formula V 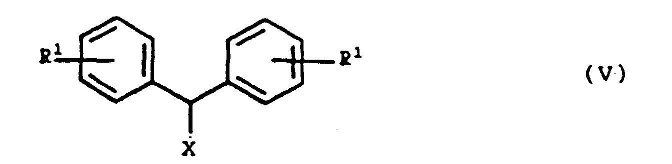 where R¹ has the meaning mentioned above, and X means hydroxy or halogen, applying standard methods of ether synthesis, consisting in a condensation reaction with the removal of water or hydrogen halide; or
c) reacting a compound of the formula IV 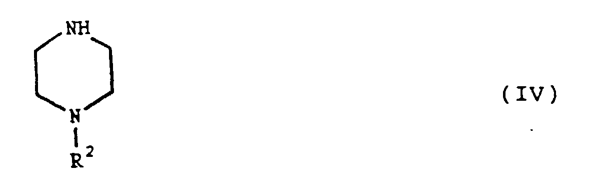 wherein R² has the meaning defined above, with an optionally substituted diarylmethoxyethyl derivative of formula VI 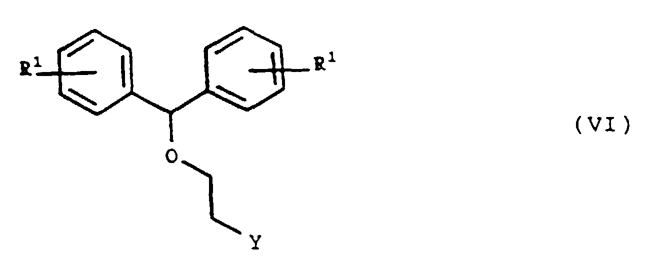 where R¹ has the meaning as defined above and Y means halogen to obtain the desired compound of formula I.

4. A pharmaceutical composition comprising as active component a compound according to claim 1 or a pharmaceutically acceptable salt thereof and a pharmaceutically acceptable carrier.

5. A pharmaceutical composition according to claim 4 in the form of an oral dosage unit containing about 50-200 mg of the active compound.

6. Use of a compound according to claim 1 for the preparation of a medicament.

7. Use of a compound according to claim 1 for the preparation of a medicament for treatment of diseases related to a dopaminergic deficit.

## Claims (Claims for the following Contracting State(s): GR)

1. A disubstituted piperazine compound having the formula I: 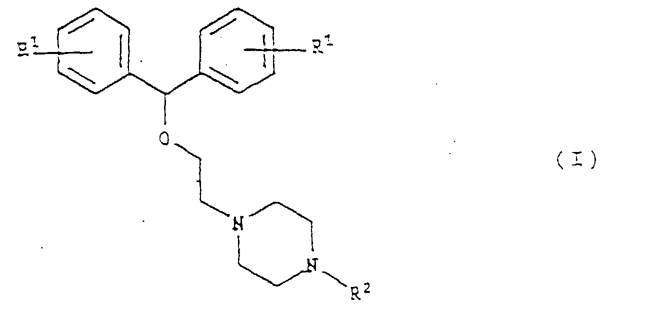 wherein R¹ is halogen, methoxy, C₁₋₆-alkyl or trifluoromethyl, and R² is a straight or branched C₁₋₈-alkyl or C₃₋₈-alkanyl, which in any position may be substituted with hydroxy-, keto-, or hydroxy imino groups leading to a stable tertiary amine, but is terminally substituted with cyano, optionally, substituted thienyl, furyl, where the optional substitution is represented by C₁₋₆-alkyl or phenyl, provided that when cyano is the only substituent in R², R² must contain at least five carbon atoms besides CN, and pharmaceutically acceptable acid addition salts thereof.

2. A compound according to claim 1 selected from the group of
1-[2-(bis(-(4-fluorophenyl)-methoxy)-ethyl]-4-(5-cyanopentyl)-piperazine or
1-[2-(bis(-(4-fluorophenyl)-methoxy)-ethyl]-4-(7-cyanoheptyl)-piperazine.

3. A method of preparing a compound according to claim 1, CHARACTERIZED in
a) reacting a compound of formula II 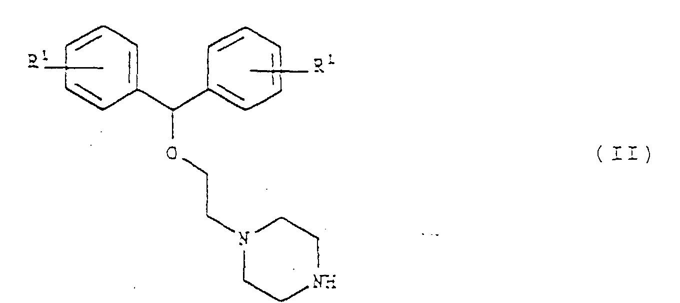 where R¹ has the meaning defined above with a reactive reagent containing the group R² as defined above, or groups that may be converted to group R²;
b) reacting a compound of formula III 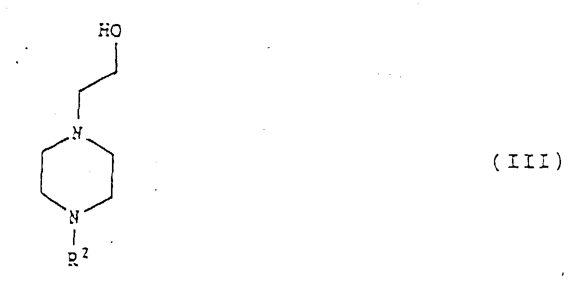 prepared by alkylation of 1-(2-hydroxyethyl)-piperazine and thereafter combining this disubstituted piperazine with a compound of formula V 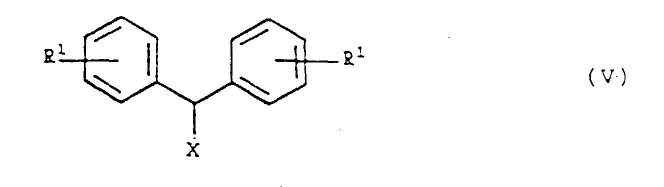 where R¹ has the meaning mentioned above, and X means hydroxy or halogen, applying standard methods of ether synthesis, consisting in a condensation reaction with the removal of water or hydrogen halide; or
c) reacting a compound of the formula IV 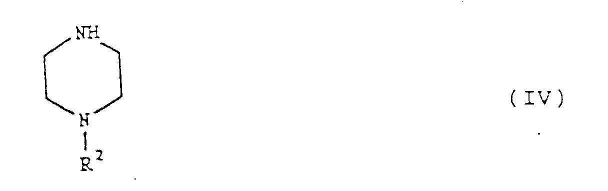 wherein R² has the meaning defined above, with an optionally substituted diarylmethoxyethyl derivative of formula VI 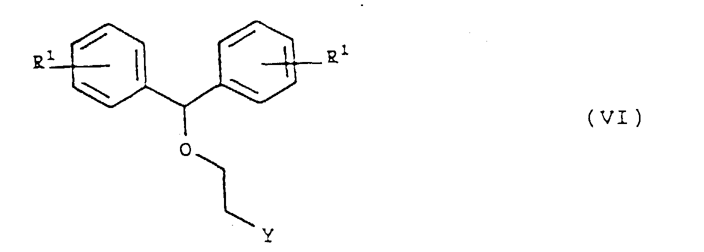 where R¹ has the meaning as defined above and Y means halogen to obtain the desired compound of formula I.

4. A process for preparing a pharmaceutical composition comprising as active component a compound according to claim 1 or a pharmaceutically acceptable salt thereof and a pharmaceutically acceptable carrier.

5. The process of claim 4 comprising formulating an oral dosage unit containing about 50-200 mg of the active compound.

6. Use of a compound according to claim 1 for the preparation of a medicament.

7. Use of a compound according to claim 1 for the preparation of a medicament for treatment of diseases related to a dopaminergic deficit.

## Claims (Claims for the following Contracting State(s): ES)

1. A method of preparing disubstituted piperazine compounds having the formula I 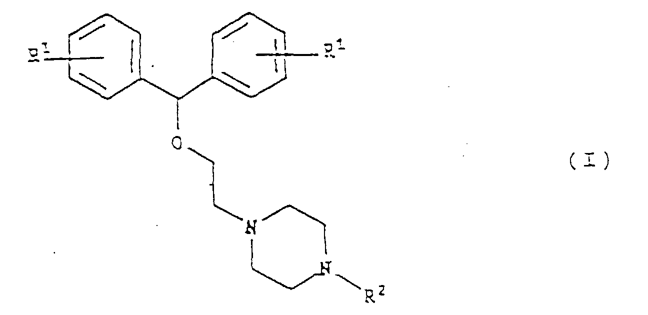 wherein R¹ is halogen, methoxy, C₁₋₆-alkyl or trifluoromethyl, and R² is a straight or branched C₁₋₈-alkyl or C₃₋₈-alkenyl, which in any position may be substituted with hydroxy-, keto-, or hydroxy imino groups leading to a stable tertiary amine, but is terminally substituted with cyano, optionally substituted thienyl, furyl, where the optional substitution is represented by C₁₋₆-alkyl or phenyl, provided that when cyano is the only substituent in R², R² must contain at least five carbon atoms besides CN, and pharmaceutically acceptable acid addition salts thereof,
CHARACTERIZED in
a) reacting a compound of formula II 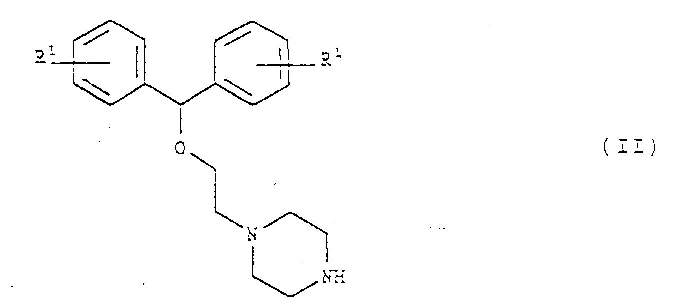 where R¹ has the meaning defined above with a reactive reagent containing the group R² as defined above, or groups that may be converted to group R²;
b) reacting a compound of formula III 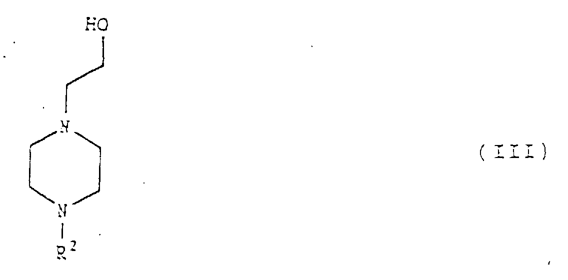 prepared by alkylation af 1-(2-hydroxyethyl)-piperazine and thereafter combining this disubstituted piperazine with a compound of formula V 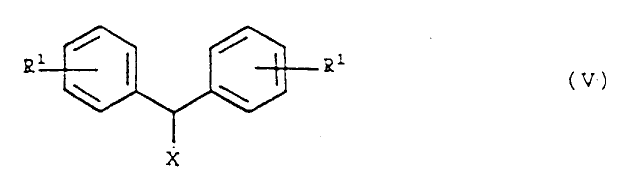 where R¹ has the meaning mentioned above, and X means hydroxy or halogen, applying standard methods of ether synthesis, consisting in a condensation reaction with the removal of water or hydrogen halide; or
c) reacting a compound of the formula IV 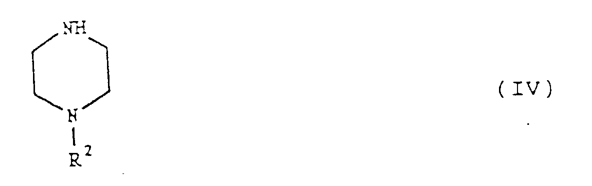 wherein R² has the meaning defined above, with an optionally substituted diarylmethoxyethyl derivative of formula VI 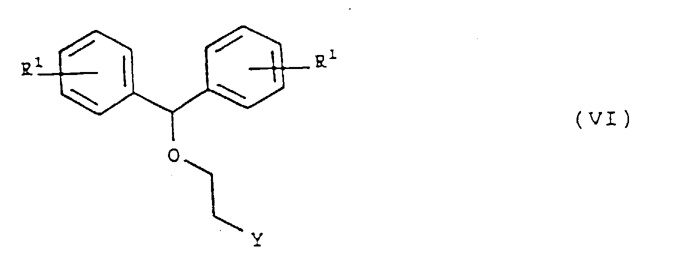 where R¹ has the meaning as defined above and Y means halogen to obtain the desired compound of formula I.

2. The compound according to claim 1 selected from the group of
1-[2-(bis(-(4-fluorophenyl)-methoxy)-ethyl]-4-(5-cyanopentyl)-piperazine or
1-[2-(bis(-(4-fluorophenyl)-methoxy)-ethyl]-4-(7-cyanoheptyl)-piperazine.

3. A process for preparing a pharmaceutical composition comprising as active component a compound according to claim 1 or a pharmaceutically acceptable salt thereof and a pharmaceutically acceptable carrier.

4. The process of claim 3 comprising formulating an oral dosage unit containing about 50-200 mg of the active compound.

5. Use of a compound prepared in the process according to claim 1 for the preparation of a medicament.

6. Use of a compound prepared in the process according to claim 1 for the preparation of a medicament for treatment of diseases related to a dopaminergic deficit.

## Patentansprüche (Patentansprüche für folgende(n) Vertragsstaat(en): AT, BE, CH, LI, DE, DK, FR GB, IT, LU, NL, SE)

1. Disubstituierte Piperazinverbindung mit der Formel I: 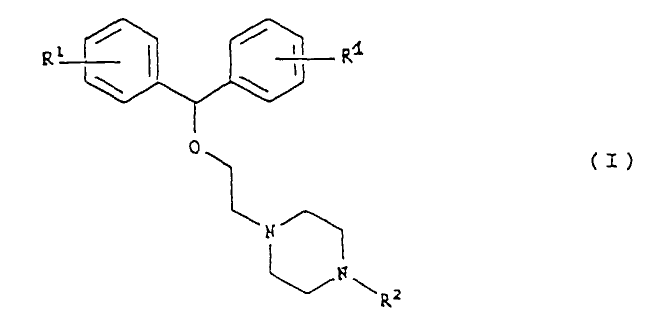 worin R¹ die Bedeutung Halogen, Methoxy, C₁₋₆-Alkyl oder Trifluormethyl hat und R² ein gerades oder verzweigtes C₁₋₈-Alkyl oder C₃₋₈-Alkenyl ist, das in einer beliebigen Position mit Hydroxy-, Keto- oder Hydroxyiminogruppen substituiert sein kann, was zu einem stabilen tertiären Amin führt, das jedoch endständig mit Cyano, wahlweise substituiertem Thienyl, Furyl, wobei die wahlweise Substitution durch C₁₋₆-Alkyl oder Phenyl dargestellt wird, substituiert ist, mit der Maßgabe, daß, wenn Cyano der einzige Substituent in R² ist, R² mindestens 5 Kohlenstoffatome neben CN enthalten muß, und pharmazeutisch verträgliche Säureadditionssalze davon.

2. Verbindung nach Anspruch 1, die aus der Gruppe aus
1-[2-(Bis-(4-fluorphenyl)-methoxy)-ethyl]-4-(5-cyanopentyl)-piperazin oder
1-[2-(Bis-(4-fluorphenyl)-methoxy)-ethyl]-4-(7-cyanoheptyl)-piperazin ausgewählt ist.

3. Verfahren zur Herstellung einer Verbindung nach Anspruch 1, gekennzeichnet durch
a) Umsetzung einer Verbindung der Formel II 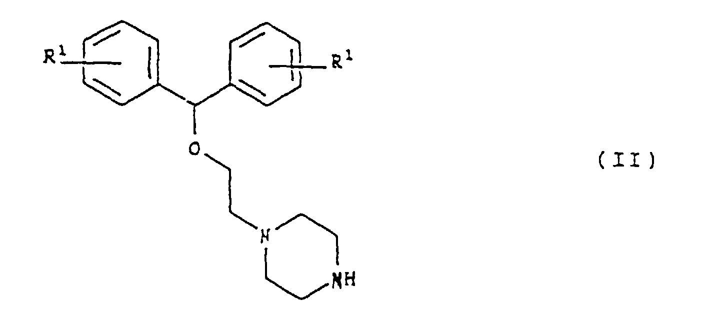 worin R¹ die vorstehend definierte Bedeutung hat, mit einem reaktien Reagenz, das die Gruppe R² gemäß der vorstehenden Definition oder Gruppen, die in die Gruppe R² umgewandelt werden können, enthält;
b) Umsetzung einer Verbindung der Formel III 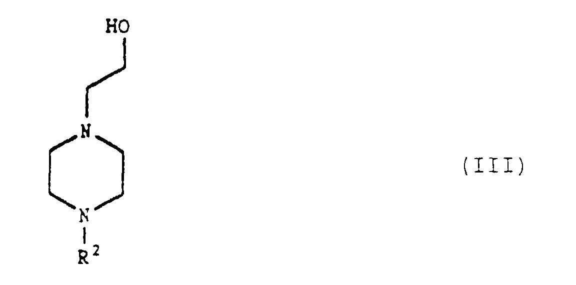 die durch Alkylierung von 1-(2-Hydroxyethyl)-piperazin hergestellt wird, und anschließende Kombination dieses disubstituierten Piperazins mit einer Verbindung der Formel V 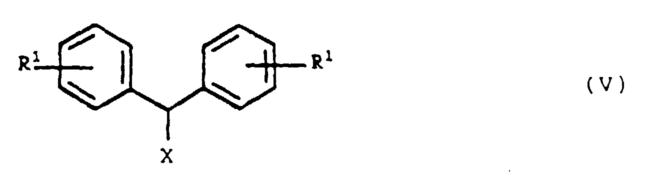 wobei R¹ die vorstehend angegebene Bedeutung hat und X die Bedeutung Hydroxy oder Halogen hat, und zwar unter Anwendung von Standardverfahren der Ethersynthese, die in einer Kondensationsreaktion mit Entfernung von Wasser oder Halogenwasserstoff bestehen; oder
c) Umsetzung einer Verbindung der Formel IV 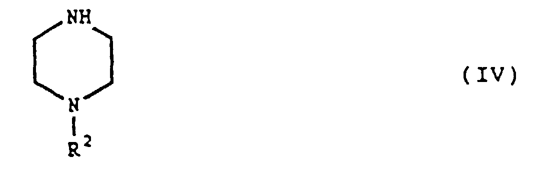 wobei R² die vorstehend definierte Bedeutung hat, mit einem wahlweise substituierten Diarylmethoxyethyl-Derivat der Formel VI 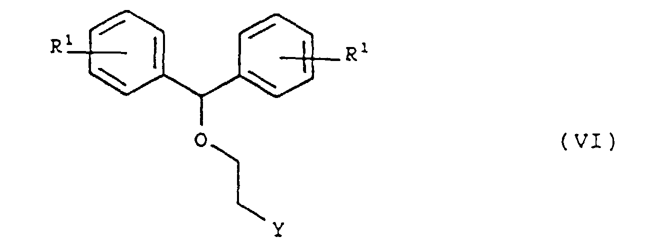 wobei R¹ die vorstehend definierte Bedeutung hat und Y Halogen bedeutet, wobei man die gewünschte Verbindung der Formel I erhält.

4. Pharmazeutische Zusammensetzung, die als aktiven Bestandteil eine Verbindung nach Anspruch 1 oder ein pharmazeutisch verträgliches Salz davon und einen pharmazeutisch verträglichen Träger enthält.

5. Pharmazeutische Zusammensetzung nach Anspruch 4 in Form einer oralen Dosiseinheit, die etwa 50-200 mg der aktiven Verbindung enthält.

6. Verwendung einer Verbindung nach Anspruch 1 für die Herstellung eines Arzneimittels.

7. Verwendung einer Verbindung nach Anspruch 1 für die Herstellung eines Arzneimittels zur Behandlung einer Erkrankung, die mit einem dopaminergen Defizit im Zusammenhang steht.

## Patentansprüche (Patentansprüche für folgende(n) Vertragsstaat(en): GR)

1. Disubstituierte Piperazinverbindung mit der Formel I: 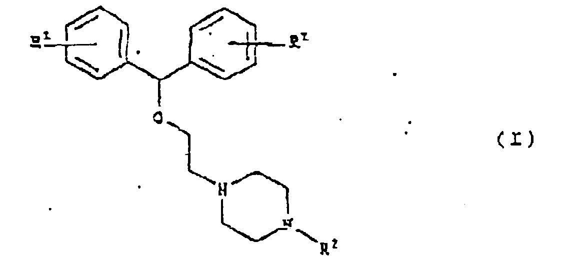 worin R¹ die Bedeutung Halogen, Methoxy, C₁₋₆-Alkyl oder Trifluormethyl hat und R² ein gerades oder verzweigtes C₁₋₈-Alkyl oder C₃₋₈-Alkenyl ist, das in einer beliebigen Position mit Hydroxy-, Keto- oder Hydroxyiminogruppen substituiert sein kann, was zu einem stabilen tertiären Amin führt, das jedoch endständig mit Cyano, wahlweise substituiertem Thienyl, Furyl, wobei die wahlweise Substitution durch C₁₋₆-Alkyl oder Phenyl dargestellt wird, substituiert ist, mit der Maßgabe, daß, wenn Cyano der einzige Substituent in R² ist, R² mindestens 5 Kohlenstoffatome neben CN enthalten muß, und pharmazeutisch verträgliche Säureadditionssalze davon.

2. Verbindung nach Anspruch 1, die aus der Gruppe
1-[2-(Bis-(4-fluorphenyl)-methoxy)-ethyl]-4-(5-cyanopentyl)-piperazin oder
1-[2-(Bis-(4-fluorphenyl)-methoxy)-ethyl]-4-(7-cyanoheptyl)-piperazin ausgewählt ist.

3. Verfahren zur Herstellung einer Verbindung nach Anspruch 1, gekennzeichnet durch
a) Umsetzung einer Verbindung der Formel II 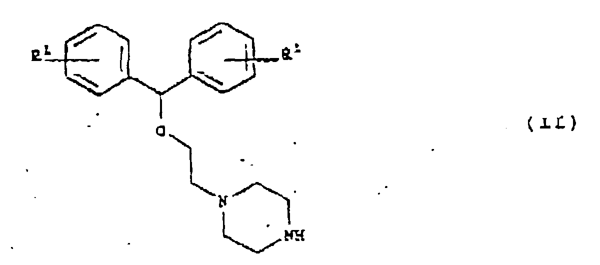 worin R¹ die vorstehend definierte Bedeutung hat, mit einem reaktien Reagenz, das die Gruppe R² gemäß der vorstehenden Definition oder Gruppen, die in die Gruppe R² umgewandelt werden können, enthält;
b) Umsetzung einer Verbindung der Formel III 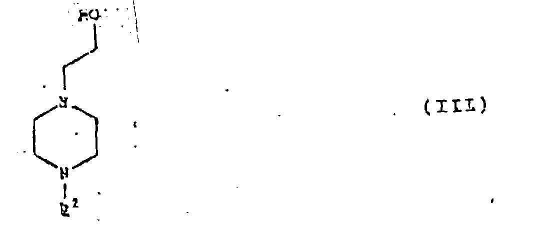 die durch Alkylierung von 1-(2-Hydroxyethyl)-piperazin hergestellt wird, und anschließende Kombination dieses disubstituierten Piperazins mit einer Verbindung der Formel V 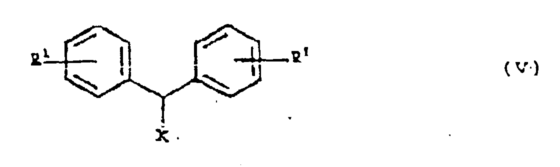 wobei R¹ die vorstehend angegebene Bedeutung hat und X die Bedeutung Hydroxy oder Halogen hat, und zwar unter Anwendung von Standardverfahren der Ethersynthese, die in einer Kondensationsreaktion mit Entfernung von Wasser oder Halogenwasserstoff bestehen; oder
c) Umsetzung einer Verbindung der Formel IV 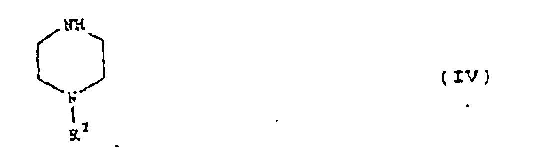 wobei R² die vorstehend definierte Bedeutung hat, mit einem wahlweise substituierten Diarylmethoxyethyl-Derivat der Formel VI 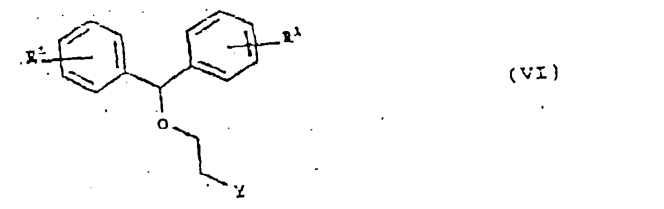 wobei R¹ die vorstehend definierte Bedeutung hat und Y Halogen bedeutet, wobei man die gewünschte Verbindung der Formel I erhält.

4. Verfahren zur Herstellung einer pharmazeutischen Zusammensetzung, die als aktiven Bestandteil eine Verbindung nach Anspruch 1 oder ein pharmazeutisch verträgliches Salz davon und einen pharmazeutisch verträglichen Träger enthält.

5. Verfahren nach Anspruch 4, umfassend die Zubereitung einer oralen Dosiseinheit, die etwa 50-200 mg der aktiven Verbindung enthält.

6. Verwendung einer Verbindung nach Anspruch 1 für die Herstellung eines Arzneimittels.

7. Verwendung einer Verbindung nach Anspruch 1 für die Herstellung eines Arzneimittels zur Behandlung von Erkrankungen, die mit einem dopaminergen Defizit im Zusammenhang stehen.

## Patentansprüche (Patentansprüche für folgende(n) Vertragsstaat(en): ES)

1. Verfahren zur Herstellung einer disubstituierten Piperazinverbindung mit der Formel I: 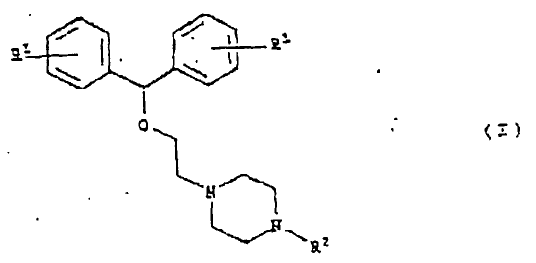 worin R¹ die Bedeutung Halogen, Methoxy, C₁₋₆-Alkyl oder Trifluormethyl hat und R² ein gerades oder verzweigtes C₁₋₈-Alkyl oder C₃₋₈-Alkenyl ist, das in einer beliebigen Position mit Hydroxy-, Keto- oder Hydroxyiminogruppen substituiert sein kann, was zu einem stabilen tertiären Amin führt, das jedoch endständig mit Cyano, wahlweise substituiertem Thienyl, Furyl, wobei die wahlweise Substitution durch C₁₋₆-Alkyl oder Phenyl dargestellt wird, substituiert ist, mit der Maßgabe, daß, wenn Cyano der einzige Substituent in R² ist, R² mindestens 5 Kohlenstoffatome neben CN enthalten muß, und pharmazeutisch verträglicher Säureadditionssalze davon,
gekennzeichnet durch
a) Umsetzung einer Verbindung der Formel II 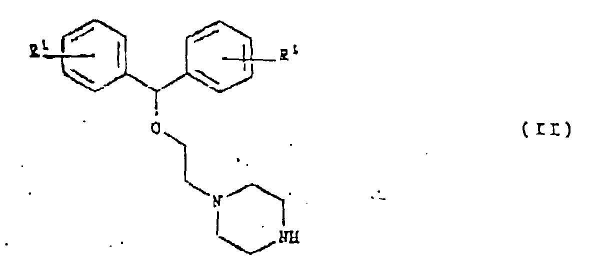 worin R¹ die vorstehend definierte Bedeutung hat, mit einem reaktien Reagenz, das die Gruppe R² gemäß der vorstehenden Definition oder Gruppen, die in die Gruppe R² umgewandelt werden können, enthält;
b) Umsetzung einer Verbindung der Formel III 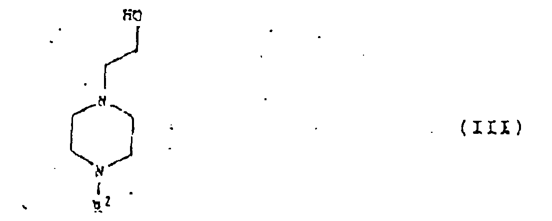 die durch Alkylierung von 1-(2-Hydroxyethyl)-piperazin hergestellt wird, und anschließende Kombination dieses disubstituierten Piperazins mit einer Verbindung der Formel V 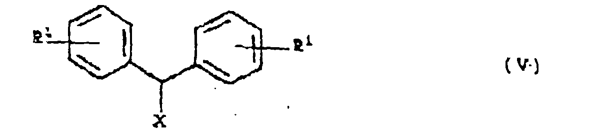 wobei R¹ die vorstehend angegebene Bedeutung hat und X die Bedeutung Hydroxy oder Halogen hat, und zwar unter Anwendung von Standardverfahren der Ethersynthese, die in einer Kondensationsreaktion mit Entfernung von Wasser oder Halogenwasserstoff bestehen; oder
c) Umsetzung einer Verbindung der Formel IV 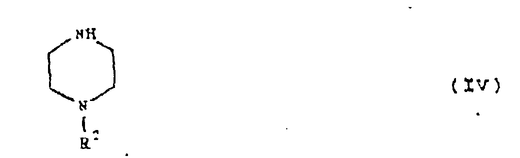 wobei R² die vorstehend definierte Bedeutung hat, mit einem wahlweise substituierten Diarylmethoxyethyl-Derivat der Formel VI 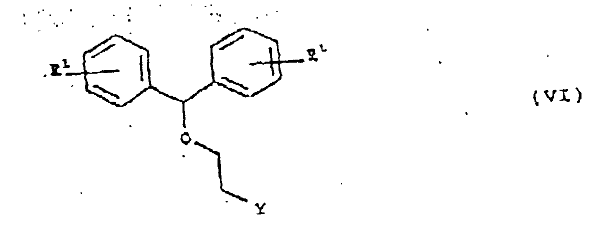 wobei R¹ die vorstehend definierte Bedeutung hat und Y Halogen bedeutet, wobei man die gewünschte Verbindung der Formel I erhält.

2. Verbindung nach Anspruch 1, die aus der Gruppe
1-[2-(Bis-(4-fluorphenyl)-methoxy)-ethyl]-4-(5-cyanopentyl)-piperazin oder
1-[2-(Bis-(4-fluorphenyl)-methoxy)-ethyl]-4-(7-cyanoheptyl)-piperazin ausgewählt ist.

3. Verfahren zur Herstellung einer pharmazeutischen Zusammensetzung, die als aktiven Bestandteil eine Verbindung nach Anspruch 1 oder ein pharmazeutisch verträgliches Salz davon und einen pharmazeutisch verträglichen Träger enthält.

4. Verfahren nach Anspruch 3, umfassend die Zubereitung einer oralen Dosiseinheit, die etwa 50-200 mg der aktiven Verbindung enthält.

5. Verwendung einer nach dem Verfahren von Anspruch 1 hergestellten Verbindung für die Herstellung eines Arzneimittels.

6. Verwendung einer nach dem Verfahren von Anspruch 1 hergestellten Verbindung für die Herstellung eines Arzneimittels zur Behandlung von Erkrankungen, die mit einem dopaminergen Defizit im Zusammenhang steht.

## Revendications (Revendications pour l'(les) Etat(s) contractant(s) suivant(s): AT, BE, CH, LI, DE, DK, FR GB, IT, LU, NL, SE)

1. Composé à base de pipérazine di-substituée ayant la formule I 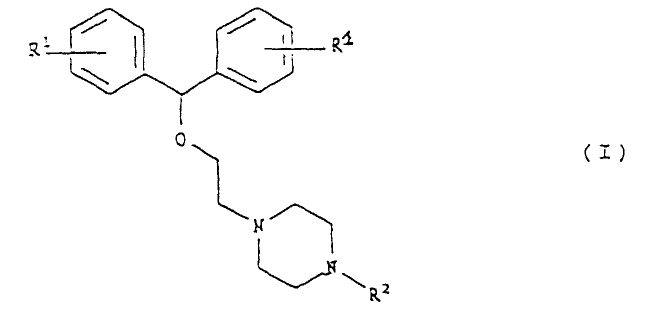 dans laquelle
R¹ est un halogène, un méthoxy, un alkyle de type C₁₋₆ ou un trifluorométhyle, et R² est un alkyle de type C₁₋₈ ou un alkényle de type C₃₋₈ linéaire ou ramifié, qui en toute position peut être substitué par des groupes hydroxy, céto, ou hydroxyimine aboutissant à une amine tertiaire stable, mais est substitué de manière terminale par un cyano, substitué de manière optionnelle par un thiényle, furyle, où la substitution optionnelle est représentée par un alkyle de type C₁₋₆ ou un phényle, pourvu que lorsque le cyano est le seul substituant en R², R² doit contenir au moins cinq atomes de carbone en plus du CN, et des sels d'addition acide de ceux-ci pouvant être acceptés de manière pharmaceutique.

2. Composé selon la revendication 1, sélectionné parmi le groupe constitué de
1-[2-(bis-(4-fluorophényl)méthoxy)éthyl]-4-(5-cyanopentyl)-pipérazine ou
1-[2-(bis-(4-fluorophényl)méthoxy)éthyl]-4-(7-cyanoheptyl)-pipérazine.

3. Procédé de préparation d'un composé selon la revendication 1, CARACTERISE par
a) la réaction d'un composé de la formule II 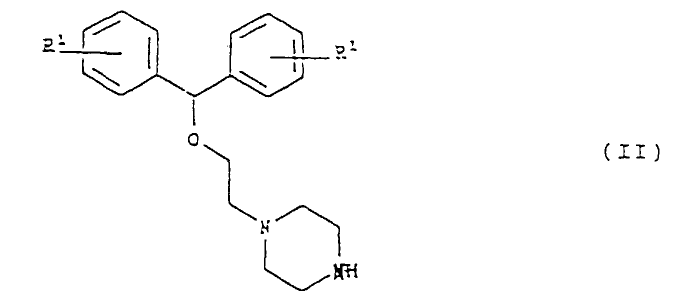 où R¹ a la signification définie ci-dessus avec un réactif chimique contenant le groupe R² tel que défini ci-dessus, ou des groupes qui peuvent être convertis en groupe R² ;
b) la réaction d'un composé de la formule III 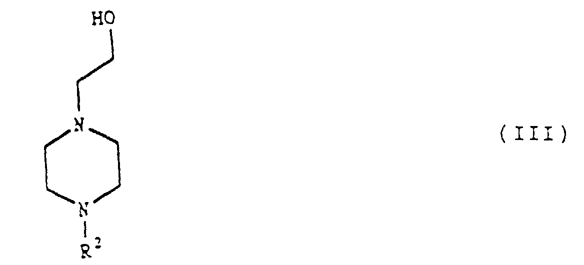 préparé par alkylation de la 1-(2-hydroxyéthyl)-pipérazine, et après quoi la combinaison de la pipérazine di-substituée avec un composé de la formule V 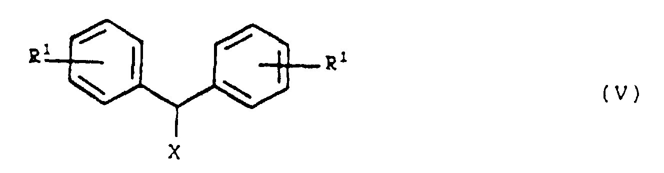 où R¹ a la signification mentionnée ci-dessus, et X est un hydroxy ou un halogène, en appliquant des procédés standards de la synthèse d'éther, consistant en une réaction de condensation avec élimination d'eau ou d'acide halohydrique,
c) la réaction d'un composé de la formule IV 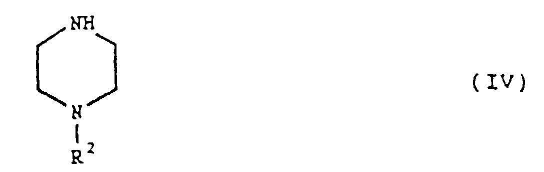 où R² a la signification telle que définie ci-dessus, ayant un dérivé diarylméthoxyéthylique de la formule VI substitué de manière optionnelle 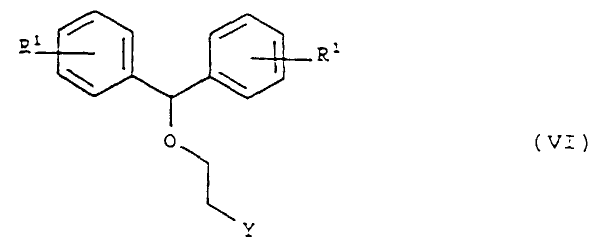 où R¹ a la signification telle que définie ci-dessus et Y est un halogène pour obtenir le composé voulu de la formule I.

4. Composition pharmaceutique comportant en tant que composant actif un composé selon la revendication 1 ou un sel de celui-ci pouvant être accepté de manière pharmaceutique et un support pouvant être accepté de manière pharmaceutique.

5. Composition pharmaceutique selon la revendication 4, sous la forme d'un dosage oral unitaire contenant environ de 50 à 200 mg du composé actif.

6. Utilisation d'un composé selon la revendication 1, pour la préparation d'un médicament.

7. Utilisation d'un composé selon la revendication 1, pour la préparation d'un médicament pour le traitement de maladies apparentées à un déficit dopaminergique.

## Revendications (Revendications pour l'(les) Etat(s) contractant(s) suivant(s): GR)

1. Composé à base de pipérazine di-substituée ayant la formule I 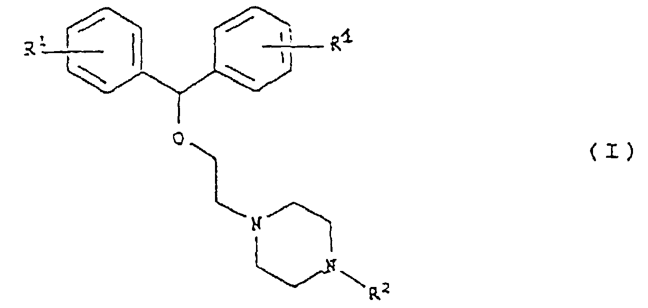 dans laquelle R¹ est un halogène, un méthoxy, un alkyle de type C₁₋₆ ou un trifluorométhyle, et R² est un alkyle de type C₁₋₈ ou un alkényle de type C₃₋₈ linéaire ou ramifié, qui en toute position peut être substitué par des groupes hydroxy, céto, ou hydroxyimine aboutissant à une amine tertiaire stable, mais est substitué de manière terminale par un cyano, substitué de manière optionnelle par un thiényle, furyle, où la substitution optionnelle est représentée par un alkyle de type C₁₋₆ ou un phényle, pourvu que lorsque le cyano est le seul substituant en R², R² doit contenir au moins cinq atomes de carbone en plus du CN, et des sels d'addition acide de ceux-ci pouvant être acceptés de manière pharmaceutique.

2. Composé selon la revendication 1, sélectionné parmi le groupe constitué de
1-[2-(bis-(4-fluorophényl)méthoxy)éthyl]-4-(5-cyanopentyl)-pipérazine ou
1-[2-(bis-(4-fluorophényl)méthoxy)éthyl]-4-(7-cyanoheptyl)-pipérazine.

3. Procédé de préparation d'un composé selon la revendication 1, CARACTERISE par
a) la réaction d'un composé de la formule II 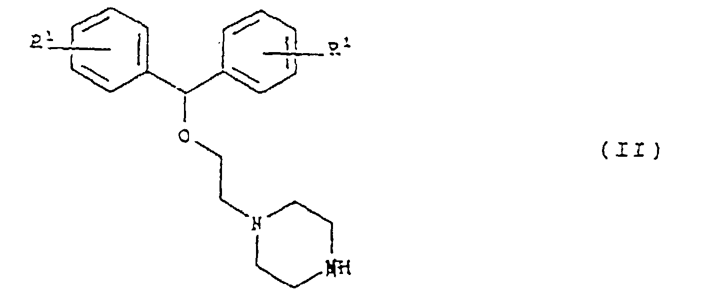 où R¹ a la signification définie ci-dessus avec un réactif chimique contenant le groupe R² tel que défini ci-dessus, ou des groupes qui peuvent être convertis en groupe R² ;
b) la réaction d'un composé de la formule III 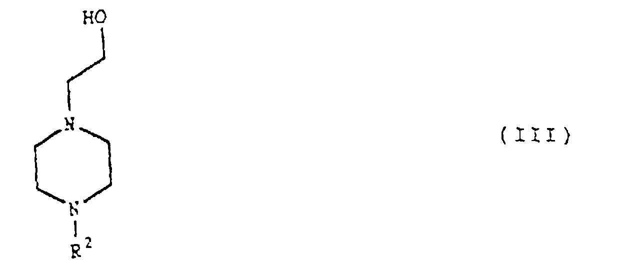 préparé par alkylation de la 1-(2-hydroxyéthyl)-pipérazine, et après quoi la combinaison de la pipérazine di-substituée avec un composé de la formule V 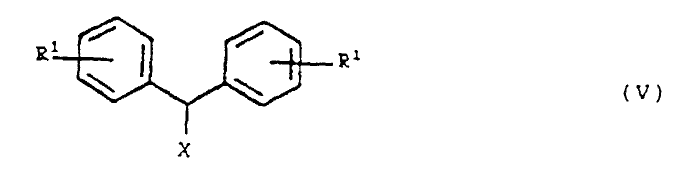 où R¹ a la signification mentionnée ci-dessus, et X est un hydroxy ou un halogène, en appliquant des procédés standards de la synthèse d'éther, consistant en une réaction de condensation avec élimination d'eau ou d'acide halohydrique,
c) la réaction d'un composé de la formule IV 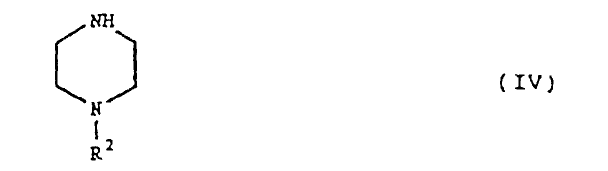 où R² a la signification telle que définie ci-dessus, ayant un dérivé diarylméthoxyéthylique de la formule VI substitué de manière optionnelle 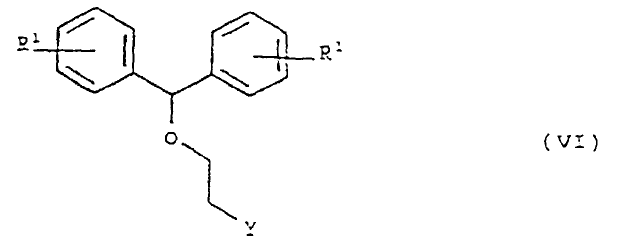 où R¹ a la signification telle que définie ci-dessus et Y est un halogène pour obtenir le composé voulu de la formule I.

4. Procédé de préparation d'une composition pharmaceutique comportant en tant que composant actif un composé selon la revendication 1 ou un sel de celui-ci pouvant être accepté de manière pharmaceutique et un support pouvant être accepté de manière pharmaceutique.

5. Procédé selon la revendication 4, consistant à réaliser un dosage oral unitaire contenant environ de 50 à 200 mg du composé actif.

6. Utilisation d'un composé selon la revendication 1, pour la préparation d'un médicament.

7. Utilisation d'un composé selon la revendication 1, pour la préparation d'un médicament pour le traitement de maladies apparentées à un déficit dopaminergique.

## Revendications (Revendications pour l'(les) Etat(s) contractant(s) suivant(s): ES)

1. Procédé de préparation de composés à base de pipérazine di-substituée ayant la formule I 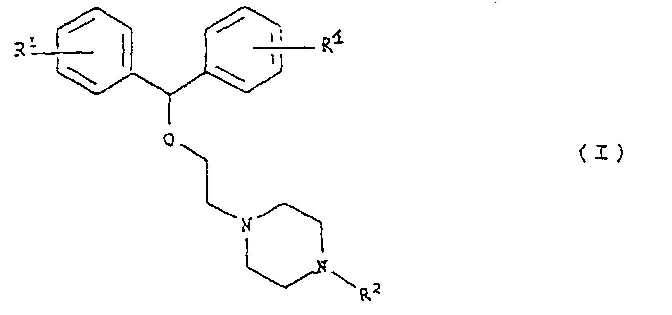 dans laquelle
R¹ est un halogène, un méthoxy, un alkyle de type C₁₋₆ ou un trifluorométhyle, et R² est un alkyle de type C₁₋₈ ou un alkényle de type C₃₋₈ linéaire ou ramifié, qui en toute position peut être substitué par des groupes hydroxy, céto, ou hydroxyimine aboutissant à une amine tertiaire stable, mais est substitué de manière terminale par un cyano, substitué de manière optionnelle par un thiényle, furyle, où la substitution optionnelle est représentée par un alkyle de type C₁₋₆ ou un phényle, pourvu que lorsque le cyano est le seul substituant en R², R² doit contenir au moins cinq atomes de carbone en plus du CN, et des sels d'addition acide de ceux-ci pouvant être acceptés de manière pharmaceutique,
CARACTERISE par
a) la réaction d'un composé de la formule II 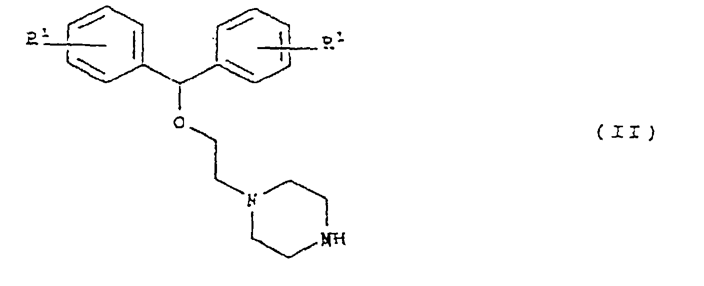 où R¹ a la signification définie ci-dessus avec un réactif chimique contenant le groupe R² tel que défini ci-dessus, ou des groupes qui peuvent être convertis en groupe R² ;
b) la réaction d'un composé de la formule III 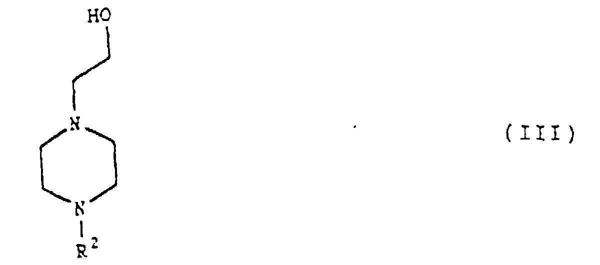 préparé par alkylation de la 1-(2-hydroxyéthyl)-pipérazine, et après quoi la combinaison de la pipérazine di-substituée avec un composé de la formule V 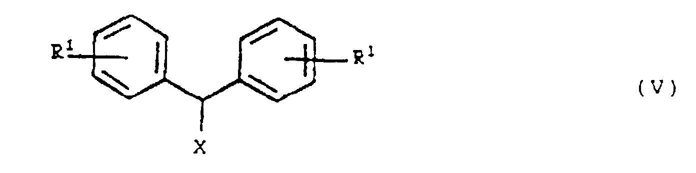 où R¹ a la signification mentionnée ci-dessus, et X est un hydroxy ou un halogène, en appliquant des procédés standards de la synthèse d'éther, consistant en une réaction de condensation avec élimination d'eau ou d'acide halohydrique,
c) la réaction d'un composé de la formule IV 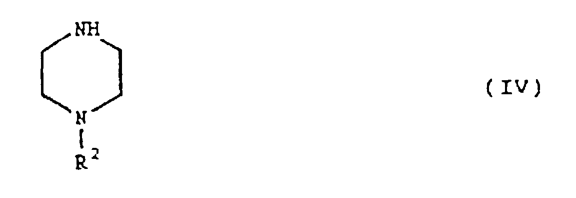 où R² a la signification telle que définie ci-dessus, ayant un dérivé diarylméthoxyéthylique de la formule VI substitué de manière optionnelle 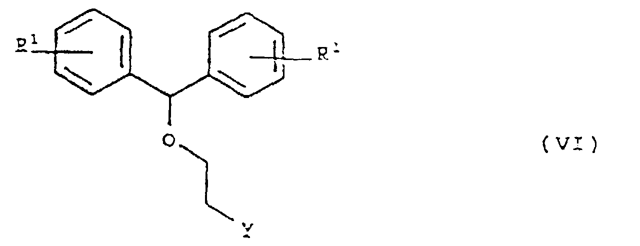 où R¹ a la signification telle que définie ci-dessus et Y est un halogène pour obtenir le composé voulu de la formule I.

2. Composé selon la revendication 1, sélectionné parmi le groupe constitué de
1-[2-(bis-(4-fluorophényl)méthoxy)éthyl]-4-(5-cyanopentyl)-pipérazine ou
1-[2-(bis-(4-fluorophényl)méthoxy)éthyl]-4-(7-cyanoheptyl)-pipérazine.

3. Procédé de préparation d'une composition pharmaceutique comportant en tant que composant actif un composé selon la revendication 1 ou un sel de celui-ci pouvant être accepté de manière pharmaceutique et un support pouvant être accepté de manière pharmaceutique.

4. Procédé selon la revendication 3, consistant à réaliser un dosage oral unitaire contenant environ de 50 à 200 mg du composé actif.

5. Utilisation d'un composé préparé dans le procédé selon la revendication 1, pour la préparation d'un médicament.

6. Utilisation d'un composé préparé dans le procédé selon la revendication 1, pour la préparation d'un médicament pour le traitement de maladies apparentées à un déficit dopaminergique.
